# EUROPEAN PATENT APPLICATION

(11) **EP 4 427 717 A1**
(43) Date of publication of application: **11.09.2024**
(21) Application number: 23160582.5
(22) Date of filing: 07.03.2023
(51) Int. Cl.: A61F 13/49, A61F 13/505, A61F 13/15

(54) **DISPOSABLE INSERT WITH FASTENER FOR RE-USABLE SHELL**

(71) Applicant: Ontex BV, 9255 Buggenhout (BE); Ontex Group NV, 9320 Erembodegem (BE)
(72) Inventor: Idelson, Alissa, 53359 Rheinbach (DE); Lambertz, Christina, 56566 Neuwied (DE)
(74) Representative: Macchetta, Andrea

(57) **Abstract**

A disposable absorbent insert comprising: a liquid permeable topsheet; a liquid impermeable backsheet; and an absorbent core comprising absorbent material therein and being sandwiched between said topsheet and backsheet; wherein the insert comprises a perimeter formed by first and second transverse edges and first and second longitudinal edges connecting the first and second transverse edges; a longitudinal centerline extending substantially parallel to said first and second longitudinal edges and interposed therebetween such to divide said insert into a first longitudinal half between said longitudinal centerline and said first longitudinal edge and a second longitudinal half between said longitudinal centerline and said second longitudinal edge; and a transverse centerline extending substantially parallel to said first and second transverse edges and interposed therebetween such to divide said insert into a first transverse half between said transverse centerline and said first transverse edge and a second transverse half between said transverse centerline and said second transverse edge; wherein the insert comprises at least one first fastening element at a body-facing side of said insert, said at least one first fastening element being positioned at least proximal to the first and/or second transverse edges; and wherein each said first fastening element is configured to secure onto a corresponding second fastening element of a re-usable outer shell.

## Description

### TECHNICAL FIELD

The disclosure relates to absorbent articles such as disposable absorbent articles, preferably selected from the group consisting of diapers (whether for baby or adults), pants (whether for baby or adults), briefs, and combinations thereof generally of the hybrid type i.e. a re-usable and/or washable outer shell and a disposable (generally single-use) absorbent insert cooperable with said outer shell.

### BACKGROUND

Hybrid-type articles, comprising a re-usable outer shell and disposable absorbent insert, are becoming more and more sought after in the market in view of their sustainable benefits and reduced waste production.

Different designs are available for such products such as described in EP 3 842 017 A1 wherein the outer shell is generally a pant-type component that may comprise elastic components therein and the disposable absorbent insert comprises liquid permeable topsheet, liquid impermeable backsheet and absorbent core sandwiched therebetween and is generally re-fastenably joinable to a crotch region of the outer shell.

Other examples are described in EP 3 659 563 A1 where the disposable insert is re-fastenably joined to the outer shell and the outer shell is in the form of a re-fastenable diaper pant; and EP 3 895 673 A1 wherein the disposable inserts are biodegradable and/or compostable.

Thus, although there have already been significant advancements in this field, there still remains a need to further improve hybrid-type articles so as to provide improved convenience of use such as ease of assembly by the user whilst keeping the design as simple as possible to permit a greater use of environmentally-friendly materials to improve the overall sustainability of the article, as well as providing a secure fit to ensure the insert does not displace during active wearing of the article and yet remain flexible enough to minimise obstruction to movement.

### SUMMARY

In a first aspect, the disclosure relates to a disposable absorbent insert comprising: a liquid permeable topsheet; a liquid impermeable backsheet; and an absorbent core comprising absorbent material therein and being sandwiched between said topsheet and backsheet; wherein the insert comprises a perimeter formed by first and second transverse edges and first and second longitudinal edges connecting the first and second transverse edges; a longitudinal centerline extending substantially parallel to said first and second longitudinal edges and interposed therebetween such to divide said insert into a first longitudinal half between said longitudinal centerline and said first longitudinal edge and a second longitudinal half between said longitudinal centerline and said second longitudinal edge; and a transverse centerline extending substantially parallel to said first and second transverse edges and interposed therebetween such to divide said insert into a first transverse half between said transverse centerline and said first transverse edge and a second transverse half between said transverse centerline and said second transverse edge; wherein the insert comprises at least one first fastening element at a body-facing side of said insert, said at least one first fastening element being positioned at least proximal to the first and/or second transverse edges; and wherein each said first fastening element is configured to secure onto a corresponding second fastening element of a re-usable outer shell.

In a second aspect, the disclosure relates to absorbent assembly comprising a disposable absorbent insert and a re-usable outer shell.

In a further aspect, the disclosure relates to a kit of parts comprising a plurality of disposable absorbent inserts and one or more re-usable outer shells.

In a further aspect, the disclosure relates to a method for the manufacture of disposable absorbent inserts, said method comprising the steps of: (i) providing an absorbent core comprising absorbent material therein; (ii) laminating the absorbent core between a first web of material and a second web and/or film of material, wherein the first web of material forms a topsheet and the second web and/or film of material forms a backsheet; (iii) providing a third web of material, preferably comprising a loop material or a nonwoven comprising an embossment pattern; and (iv) applying said third web of material over at least portions of the first web of material corresponding to positions at least proximal to first and/or second transverse edges of the insert to form least one first fastening element.

### BRIEF DESCRIPTION OF THE FIGURES

**Fig. 1A-C** Illustrate, top planar view (topsheet-side), of inserts according to embodiments herein.
**Fig. 2** Illustrates, a top planar view (topsheet-side), of an insert in an un-folded state according to an embodiment herein.
**Fig. 3** Illustrates, a top planar view (topsheet-side), of an insert in a folded state according to an embodiment herein.
**Fig. 4** Illustrates, a top planar view (topsheet-side), of an insert in a folded state according to an embodiment herein.
**Fig. 5** Illustrates, a cross-section, of an insert in a folded state according to an embodiment herein.
**Fig. 6A-C** Illustrate, cross-sections, of inserts according to embodiments herein.
**Fig. 7** Illustrates, a top planar view (body-facing side), of a re-usable outer shell according to an embodiment herein.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.
"About" or "substantially" as used herein referring to a measurable value such as a parameter, an amount, a temporal duration, and the like, is meant to encompass variations of +/-20% or less, preferably +/-10% or less, more preferably +/-5% or less, even more preferably +/-1 % or less, and still more preferably +/-0.1 % or less of and from the specified value, in so far such variations are appropriate to perform in the disclosed disclosure. However, it is to be understood that the value to which the modifier "about" or "substantially" refers is itself also specifically disclosed.
"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.
The expression "% by weight" or "%wt" (weight percent), here and throughout the description unless otherwise defined, refers to the relative weight of the respective component based on the overall weight of the formulation.
The use of the term "layer" can refer, but is not limited, to any type of substrate, such as a woven web, nonwoven web, films, laminates, composites, elastomeric materials, absorbent materials (such as SAP and cellulose fibers/fluff mixtures), or the like. A layer can be liquid and air permeable, permeable to air but impermeable to liquids, impermeable both to air and liquid, or the like. When used in the singular, it can have the dual meaning of a single element or a plurality of elements, such as a laminate or stacked plural sub-layers forming a common layer.
The terms "nonwoven", "nonwoven layer" or "nonwoven web" are used interchangeably to mean an engineered fibrous assembly, primarily planar, which has been given a designed level of structural integrity by physical and/or chemical means, excluding weaving, knitting or papermaking (ISO 9092:2019 definition). The directionally or randomly orientated fibers, are bonded by friction, and/or cohesion and/or adhesion. The fibers may be of natural or synthetic origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter (g/m2 or gsm).
The term "disposable" refers to absorbent articles and/or inserts that generally are not intended to be laundered or otherwise restored or reused as absorbent articles, i.e., they are intended to be discarded after a single use and, preferably, to be recycled, composted or otherwise disposed of in an environmentally compatible manner.
The term "disposed" is used to mean that an element(s) is formed (joined and positioned) in a particular place or position as a unitary structure with other elements or as a separate element joined to another element.
The terms "interior" and "exterior" refer respectively to the location of an element that is intended to be placed against or toward the body of a wearer when an absorbent article is worn and the location of an element that is intended to be placed against or toward any clothing that is worn over the absorbent article. Synonyms for "interior" and "exterior" include, respectively, "inner" and "outer", as well as "inside" and "outside", or "body-facing" and "garment-facing". Also, when the absorbent article is oriented such that its interior faces upward, e.g., when it is laid out in preparation for setting the wearer on top of it, synonyms include "upper" and "lower" and "top" and "bottom", respectively.
The term "joined" refers to configurations whereby an element is directly secured to another element by attaching the element directly to the other element, and configurations whereby an element is indirectly secured to another element by attaching the element to intermediate member(s) which in turn are attached to the other element.
The term "lateral" or "transverse" refers to a direction running at a 90 degree angle to the longitudinal direction and when combined with the term "substantially" includes directions within ±45° of the lateral direction.
The term "longitudinal" refers to a direction running parallel to the maximum linear dimension of the article and when combined with the term "substantially" includes directions within ±45° of the longitudinal direction.
"Plant-based fibers", as used herein, includes both harvested fibers and synthetic fibers that comprise bio-based content. Harvested plant-based fibers include cellulosic matter, such as wood pulp; seed hairs, such as cotton; stem (or bast) fibers, such as flax and hemp; leaf fibers, such as sisal; and husk fibers, such as coconut. Assessment of the renewably based carbon in a material can be performed through standard test methods. Using radiocarbon and isotope ratio mass spectrometry analysis, the bio-based content of materials can be determined. ASTM International has established a standard method for assessing the bio-based content of materials. The ASTM method is designated ASTM D6866-10.The application of ASTM D6866-10 to derive a bio-based content and the analysis is performed by deriving a ratio of the amount of organic radiocarbon (14C) in an unknown sample to that of a modern reference standard. The ratio is reported as a percentage with the units "pMC" (percent modern carbon). The modern reference standard used in radiocarbon dating is a NIST (National Institute of Standards and Technology) standard with a known radiocarbon content equivalent approximately to the year AD 1950. AD 1950 was chosen since it represented a time prior to thermo-nuclear weapons testing which introduced large amounts of excess radiocarbon into the atmosphere with each explosion (termed "bomb carbon"). The AD 1950 reference represents 100 pMC.
"Bio-based content" refers to the amount of carbon from a renewable resource in a material as a percent of the mass of the total organic carbon in the material, as determined by ASTM D6866- 10, method B. In order to apply the methodology of ASTM D6866-10 to determine the bio-based content of any absorbent article or component thereof, a sample can be ground into particulates less than about 20 mesh using known grinding methods (e.g., WILEY mill), and a representative sample of suitable mass taken from the randomly mixed particles. Alternatively, if the sample is merely a layer of material, then the layer itself can be analyzed without the need for a pre-grinding step. Note that any carbon from inorganic sources such as calcium carbonate is not included in determining the bio-based content of the material.

Embodiments of the articles according to the disclosure will now be described. It is understood that technical features described in one or more embodiments maybe combined with one or more other embodiments without departing from the intention of the disclosure and without generalization therefrom, especially when such combinations are explicitly or implicitly inferred.

### THE OUTER SHELL

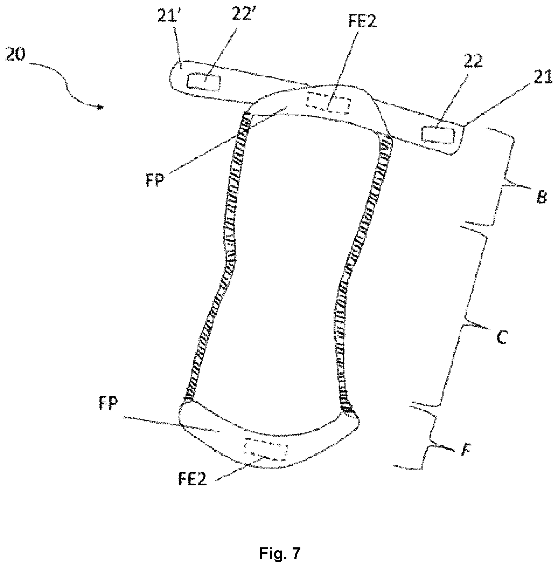

As exemplified in Fig. 7, re-usable outer shells (20) herein preferably comprise: a front (F) and back (B) waist region having uppermost and lowermost edges; and a crotch region (C) interposed between the front and back waist region (F, B), wherein the uppermost edges of the waist region form a waist opening and the lowermost edges of the waist region together with lateral extremities of the crotch region form two oppositely disposed leg openings; and wherein at least the body-facing surface of the crotch region (C) is adapted for receiving an absorbent insert (1) as described herein.

In a preferred embodiment, each of the front (F) and/or back (B) waist regions comprise a flap (FP) extending from the upper and/or lowermost edges respectively towards said crotch region (C) along an axis parallel to the longitudinal centerline (y); wherein said flap(s) (FP) overlap the one or more second fastening elements (FE2) that are positioned on a body-facing surface of said re-usable outer shell (20); or wherein said flap(s) (FP) comprise the one or more second fastening elements (FE2) on a garment facing surface thereof. Advantageously such flaps allow for protection to the skin of the wearer from hooks as well as providing optimal securing fit when cooperating with an insert having loop material on the body-facing surface thereof.

In an embodiment, at least a portion of said crotch region (C) corresponding with a wetness indicator (when present) of the insert (1) is translucent such that said wetness indicator is viewable from a garment facing side of said re-usable outer shell (20) or comprises an optical sensor adapted to automatically detect a colour change of said wetness indicator and send a corresponding signal to an application device. Advantageously this not only allows to provide effective saturation indication when using a hybrid-article but further allows for at the same time providing indicia to correctly position the insert in the right front/back relationship that may be particularly useful when designing inserts that have a non-uniform absorption profile (e.g. varying basis weight of absorbent material for optimised absorption and limited over-specked or wasted material which then require the correct front/back orientation on placement). In the latter case the insert may be correctly placed when the wetness indicator is fully/entirely viewable through the outer shell (when viewed on the garment side thereof). This may be verified either directly, i.e. visually, by a caregiver viewing the wetness indicator indicia or indirectly, by automatic identification by the sensor that may then provide a warning via an application device to a caregiver accordingly.

The re-usable outer shell (20) may comprise re-fastenable or permanent side seams joining the front and back waist regions, preferably in the form of a pant; or the back waist region (B) comprises transversely extending side panels (21, 21') each comprising a fastener (22, 22') for coupling to a landing zone positioned at a garment facing side of the front waist region (F), preferably in the form of a diaper.

The fastener (22, 22') may be in the form of hooks. The outer shell (20) may comprise a first hook member (22) in the rear region provided in a first rear transverse corner region of the shell (20) [or first side panel (21)] and a second hook member (22') provided in a second rear transverse corner region of the shell (20) [or second side panel (21')]. The outer shell (20) comprises a hook attachment zone on the outer surface in the front region of the outer shell (not shown).

The hook attachment zone may comprise a loop material. The first (22) and second (22') hook members are adapted to be attached to the hook attachment zone to form a waist opening when the shell (20) is worn.

The first (22) and second (22') hook members may comprise any hook-type material with a hook surface structure and the hook attachment zone may comprise any loop surface structure, and the terms hook members are to be interpreted as encompassing the hook part of a hook and loop fastening system, e.g., a system known under the tradename VELCRO^{®}. The "hooks" may have many different shapes which are adapted to engage with a loop part of the hook and loop fastening system. The hooks may, purely as an example, have a J-shape, mushroom shape, and/or a palm tree shape. The hook attachment zone may comprise any type of loop surface structure, which is here to be understood to encompass a surface structure to which a hook material of a hook and loop fastening system is attachable. Accordingly, a loop surface structure may include fibers or threads extending from a surface and back into the surface to define genuine loops, as well as a surface structure including fibers or threads extending out from a surface and having loose ends, which fibers or threads entangle with each other. Upon attachment ofthe respective hook members the hook surface structure engages with the loop surface structure providing a releasable attachment between the elements.

Hooks or hook members described herein may generally comprise any hook-type material with a hook surface structure and the terms hook members are to be interpreted as encompassing the hook part of a hook and loop fastening system, e.g., a system known under the trade name VELCRO^{®}. The "hooks" may have many different shapes which are adapted to engage with a loop part of the hook and loop fastening system. The hooks may, purely as an example, have a J-shape, mushroom shape, and/or a palm tree shape.

In an embodiment the crotch region (C) of the re-usable outer shell (20) comprises a window (23) for viewing said wetness indicator from a garment-facing side of said shell (20). The crotch region (C) is generally adapted for receiving an absorbent insert (1) as described herein and wherein at least a portion and/or area of said crotch region comprises said window (23) that substantially corresponds with the position of the wetness indicator, when the insert (1) is joined to the shell (20). Advantageously this allows for visual inspection of saturation and/or correct positioning directly by a subject on use.

The window herein may be in the form of a cut-out or opening, or may comprise a translucent and/or transparent material such as a film or other suitable synthetic covering.

### THE INSERT

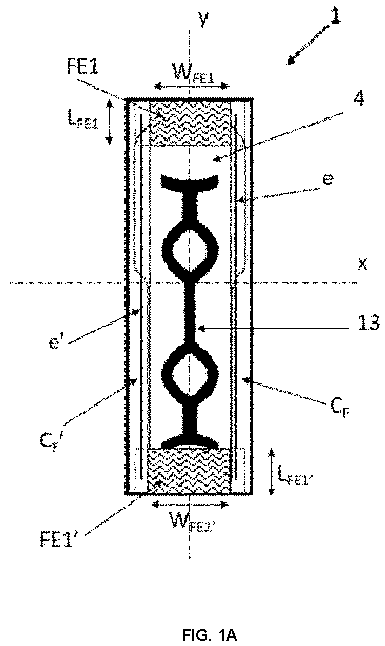

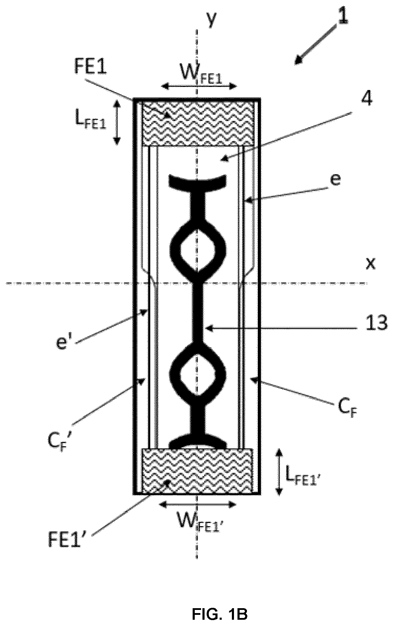

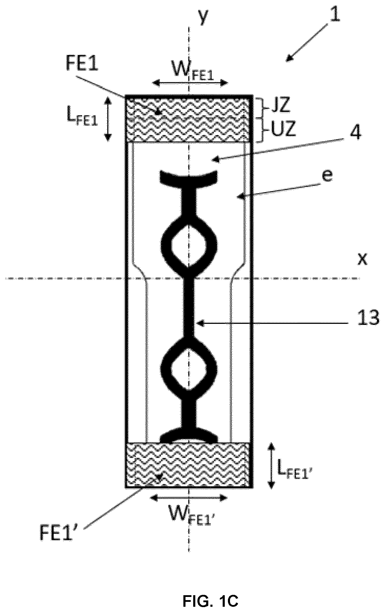

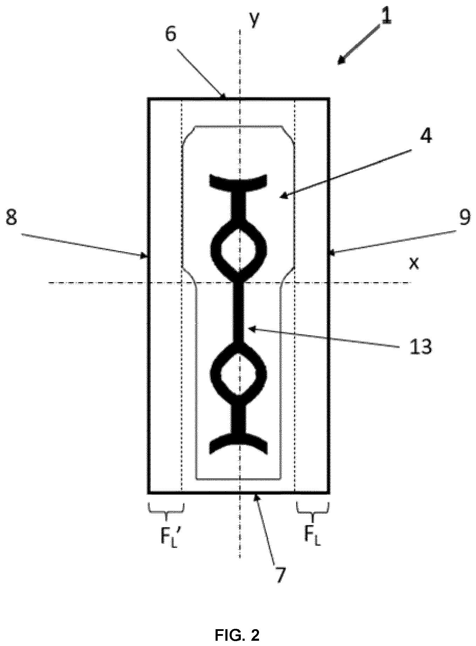

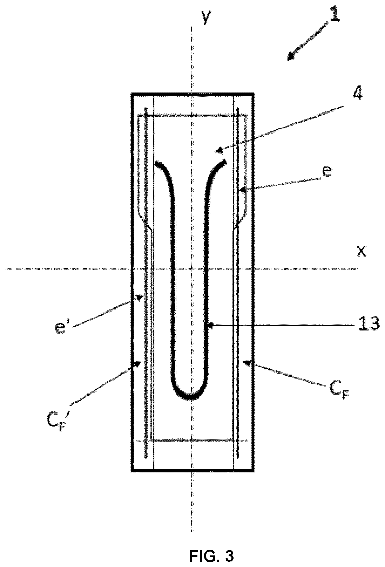

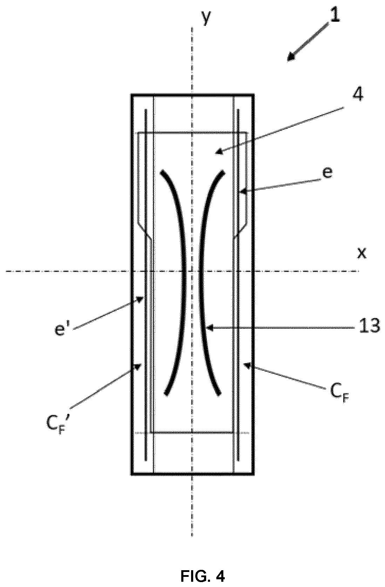

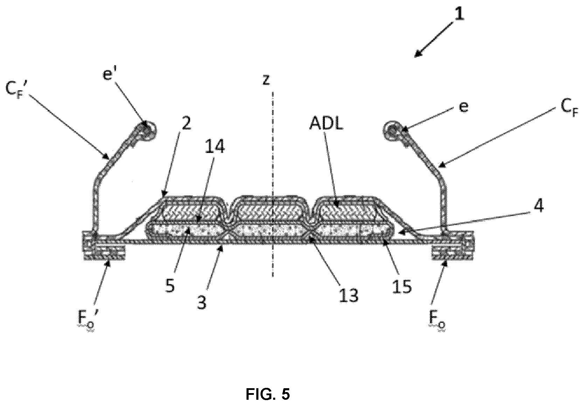

As exemplified in Figs. 1-5, inserts (1) herein typically comprise a liquid permeable topsheet (2), a liquid impermeable backsheet (3); and an absorbent core (4) comprising absorbent material (5) therein and being sandwiched between said topsheet (2) and backsheet (3). "Inserts" are generally a disposable component of an assembly comprising a re-usable outer cover. Inserts are generally different from traditional disposable diapers or pants in that they do not comprise side panels such as elastic ears or belts, or other components that are generally challenging to compost or recycle, at the same time they typically necessitate the use of a re-usable component to hold the insert in place during use.

Generally, the inserts herein comprise a perimeter formed by first and second transverse edges (6, 7) and first and second longitudinal edges (8, 9) connecting the first and second transverse edges (6, 7); a longitudinal centerline extending (y) substantially parallel to said first and second longitudinal edges (8, 9) and interposed therebetween such to divide said insert (1) into a first longitudinal half between said longitudinal centerline (y) and said first longitudinal edge (8) and a second longitudinal half between said longitudinal centerline (y) and said second longitudinal edge (9); and a transverse centerline (x) extending substantially parallel to said first and second transverse edges (6, 7) and interposed therebetween such to divide said insert (1) into a first transverse half between said transverse centerline (x) and said first transverse edge (6) and a second transverse half between said transverse centerline (x) and said second transverse edge (7).

Inserts (1) herein comprise at least one first fastening element (FE1) at a body-facing side of said insert (1), said at least one first fastening element (FE1) being positioned at least proximal, preferably adjacent, to the first and/or second transverse edges (6, 7), wherein each said first fastening element (FE1) is configured to secure onto a corresponding second fastening element (FE2) of a re-usable outer shell (20). It is understood herein that "configured to" generally means that the first fastening element(s) (FE1) are expressly and/or purposively designed for joining and/or adhering to the corresponding second fastening element (FE2). Advantageously, and counter-intuitively, by placing the fastening elements on a body-facing side of the insert it allows for reduced risk of irritation onto a subject's skin compared to fasteners located on a garment facing surface of the insert. For example, there is a reduced risk that misplacement of the insert causes hooks present on the re-usable outer shell to come into contact with the skin of the wearer. Moreover, when used in combination with shells having hooks on a body-facing surface thereof allows for a thicker padding that results from the respective portion of the insert being folded outwards to engage the fasteners together; or when used in combination with shells having hooks on a garment facing surface of a respective flap (as described in more detail herein below), permits not only to ensure the hooks are always facing away from the wearer providing the abovementioned reduced risk of irritation but further permits a more secure fit.

The first fastening element(s) (FE1) can be portions of the topsheet (2) itself. In this embodiment, the topsheet (2) is preferably a nonwoven comprising a plurality of macroscopic peaks and recesses formed by for example embossing to allow increased surface area for corresponding second fastening element(s) (e.g. hooks) to engage thereon. The embossing may comprise a repeating pattern of shapes such as waves, lines, or the like. By "macroscopic" it is intended that the pattern formed by the peaks and recesses is visible by naked eye. When portion(s) of the topsheet (2) itself forms the first fastening element(s) (FE1), such portions may be provided with a texture that is different from the rest of the topsheet (2), said texture being formed by either the embossment pattern being different in said portions compared to the rest of the topsheet (2) (for example, in density of peaks and/or protrusions per cm2) or by using a different adhesive pattern to adhere any under-layer(s) (e.g. the absorbent core) thereto, such as using a greater adhesive pattern density in such portions compared to the rest of the topsheet generally such to on the one hand limit risk of delamination and on the other hand provide a compact structure for hook engagement to limit premature release by de-fiberisation. Alternatively, or additionally, (and yet preferred) the first fastening element(s) (FE1) comprise, preferably consist of, a layer that is distinct and/or separate from the topsheet (2), and the first fastening element(s) (FE1) being preferably selected from: a nonwoven comprising a plurality of macroscopic peaks and recesses (as described above); or loop material.

The first fastening element(s) (FE1) may comprise, or consist of, a nonwoven comprising a plurality of macroscopic peaks and recesses (as described above); or loop material.

The first fastening element(s) (FE1) may be in the form of strips having a length running substantially parallel to a longitudinal centerline (y) of the insert and a width running substantially perpendicular to the longitudinal centerline (y) and substantially parallel to transverse centerline (x). The width of said first fastening element(s) (FE1) may be from 50% to 100%, preferably from 60% to 95%, even more preferably from 70% to 90%, of the total width of the insert along said transverse centerline (x), and the length of said first fastening element(s) (FE1) may be from 1% to 30%, preferably from 5% to 25%, even more preferably from 10% to 20%, of the total length of the insert along said longitudinal centerline (y).

Th loop material on the topsheet may have any type of loop surface structure, which is here to be understood to generally encompass a surface structure to which a hook material of a hook and loop fastening system is attachable. Accordingly, a loop surface structure may include fibers or threads extending from a surface and back into the surface to define genuine loops, as well as a surface structure including fibers or threads extending out from a surface and having loose ends, which fibers or threads entangle with each other. Upon attachment of the respective hook members the hook surface structure engages with the loop surface structure providing a releasable attachment between the elements. The loop material may also be a nonwoven material or a nonwoven material with a backing of an elastic film. The basis weight of the loop material may be 15-70 gsm, such as 30-60 gsm.

Preferably, the first fastening element (FE1) comprises a layer that is joined to the topsheet (2) on a body-facing surface thereof, preferably wherein said layer is joined by adhesive along a joining line extending parallel to the first and/or second transverse edges (6, 7). It is preferred that the adhesive extends substantially continuously from a first application end (e.g. positioned adjacent a first extremity of the first fastening element (FE1) being closest to the first longitudinal edge (8) of the insert) to a second application end (e.g. positioned adjacent a second extremity of the first fastening element (FE1) being closest to the second longitudinal edge (9) of the insert)

In a preferred embodiment, the insert comprises at least two first fastening elements (FE1, FE1') joined to the topsheet (2) on a body-facing surface thereof and each being joined at a position proximal, preferably adjacent, to the first or second transverse edges (6, 7) respectively such that said first fastening elements (FE1, FE1') are separated from each other at least along an axis extending substantially parallel to the longitudinal centerline (y). Advantageously this permits good anchoring of the insert to the outer shell and yet permits to save on material cost. Moreover, especially when the first fastening elements comprise a film it may further reduce hindrance to breathability.

In an embodiment, the first fastening element (FE1), preferably each first fastening element (FE1, FE1'), comprises, preferably consists of, a female fastening element; and the second fastening element (FE2) comprises, preferably consists of, a male fastening element.

Preferably, the first fastening element (FE1), preferably each first fastening element (FE1, FE1'), comprises knitted loop material or a nonwoven loop material; and/or wherein the corresponding second fastening element (FE2) comprises hooks.

Preferably, the first fastening element(s) (FE1, FE1') have a length (L_{FE1}, L_{FE1'}) extending substantially parallel to the longitudinal centerline (y) and a width (W_{FE1}, W_{FE1'}) extending substantially parallel to the transverse centerline (x), and the length-to-width ratio (L_{FE1}/W_{FE1}, L_{FE1'}/W_{FE1'}) of less than 1, preferably from 0.1 to 0.9, more preferably from 0.2 to 0.8, even more preferably from 0.3 to 0.7. Advantageously this allows for superior attachment and skin protection.

In a preferred embodiment, the inserts herein comprise a front first fastening element (FE1) and a rear first fastening element (FE1'), wherein the front first fastening element (FE1) is positioned proximal, preferably adjacent, to the first transverse edge (6) and that generally is positioned on the front waist region (F) of the re-usable outer shell (20); and wherein the rear first fastening element (FE1') is positioned proximal, preferably adjacent, to the second transverse edge (7) and that generally is positioned on the back waist region (B) of the re-usable outer shell (20). The front first fastening element (FE1) may have a length (L_{FE1}) extending substantially parallel to the longitudinal centerline (y) and the rear first fastening element (FE1') may have a length (L_{FE1'}) extending substantially parallel to the longitudinal centerline (y); and wherein L_{FE1'} is greater or equal to L_{FE1}, preferably wherein L_{FE1'} is greater than L_{FE1}, more preferably wherein L_{FE1'} is from 1.1L_{FE1} to 3L_{FE1} , even more preferably from 1.2L_{FE1} to 2.5L_{FE1}, even more preferably from 1.4L_{FE1} to 2L_{FE1}. Advantageously this allows for a sufficiently large containment pocket to be formed towards the back of the insert where found more useful whilst limiting the amount of material usage. Particularly advantageous is the asymmetric arrangement described which allows for a synergistic combination of fastening ability, exudate containment and reduced material usage.

In an embodiment, the length L_{FE1}, is from 20mm to 100mm, preferably from 30mm to 80mm, even more preferably from 35mm to 65mm. Advantageously this allows for optimal engagement surface for respective second fastening elements of the outer shell to secure thereto.

In an embodiment, the first fastening element(s) (FE1, FE1') are joined to the topsheet (2) at a joining zone (JZ) proximal, preferably adjacent, to the first and/or second transverse edges (6, 7) wherein said joining zone comprises adhesive; and wherein the first fastening element(s) (FE1, FE1') comprise an un-joined zone (UZ) substantially free of adhesive, wherein the un-joined zone (UZ) is positioned adjacent to the joining zone (JZ) and closer to the transverse centerline (x) than said joining zone (JZ). Preferably, a pocket is formed between the topsheet (2) and the first fastening element(s) (FE1, FE1') at the un-joined zone (UZ) to accommodate exudates therein. Advantageously this allows for a dual-use synergistic component that acts both as fastening to the outer shell and also as a containment pocket for exudates.

Especially in combination with the embodiment above, the garment-facing surface of the first fastening element(s) (FE1, FE1') (e.g. the one facing and/or joined to the topsheet) comprises a hydrophobic layer or a liquid impermeable film preferably in the form of a film backing onto which the loop material or nonwoven loop is applied, more preferably wherein said film is elastic. Advantageously this provides even better exudate containment and gasketing fit onto the skin of a wearer.

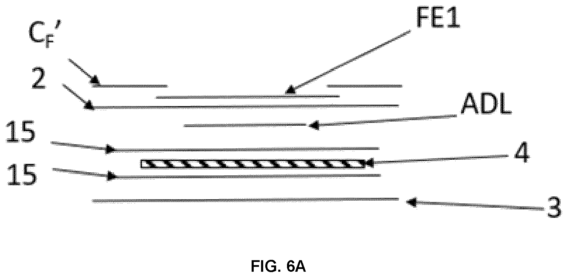

As explained in more detail herein below, and with reference to Fig. 6A-B, the inserts herein may comprise at least a pair of lateral cuffs (C_{F}, C_{F}') wherein at least one of said cuffs is positioned along at least a portion of the first longitudinal edge (8) and at least one other of said cuffs is positioned along at least a portion of the second longitudinal edge (9), preferably wherein the cuffs comprise one or more elastics (e, e') proximal to an apex thereof such that a tension thereof causes the cuffs to project upwards away from the topsheet (2); and wherein said cuffs are joined to a body-facing surface of the first fastening element(s) (FE1, FE1'), or wherein said cuffs are joined to a garment-facing surface of the first fastening element(s) (FE1, FE1') and are at least partly overlapped by said first fastening element(s) (FE1, FE1') such that said cuffs are positioned between the topsheet (2) and said first fastening element(s) (FE1, FE1'). Advantageously this allows for a reinforced region to be formed that eases folding whilst yet enabling the barrier effects (e.g. in embodiments herein-above) and yet maximises the surface area available for fastening.

In an embodiment, as shown in Fig. 6A-C, the insert comprises at least a pair of lateral cuffs (C_{F}, C_{F}') wherein at least one of said cuffs is positioned along at least a portion of the first longitudinal edge (8) and at least one other of said cuffs is positioned along at least a portion of the second longitudinal edge (9) such to define a spacing therebetween, and such that the at least one first fastening element (FE1) is exposed, preferably only, along said spacing. Preferably wherein said cuffs (C_{F}, C_{F}') are positioned on a body-facing surface of the at least one first fastening element (FE1) to define at least two regions where said cuffs (C_{F}, C_{F}') overlap said at least one first fastening element (FE1). In such a way the surface area available for fastening is maximised yet enabling superior exudate barrier properties (i.e. reduced leakage) as described in embodiments herein which is at least partly enabled by the cuffs positioned over the fastening elements in the thickness direction.

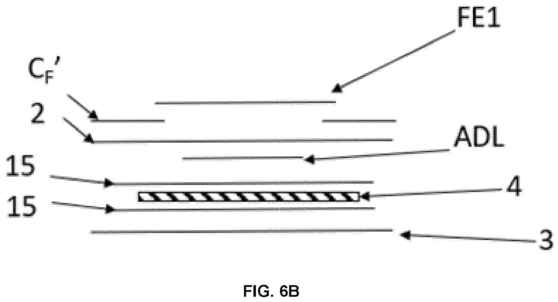

In an embodiment, as shown in Fig. 6B, the insert comprises at least a pair of lateral cuffs (C_{F}, C_{F}') wherein at least one of said cuffs is positioned along at least a portion of the first longitudinal edge (8) and at least one other of said cuffs is positioned along at least a portion of the second longitudinal edge (9) such to define a spacing therebetween, and wherein said cuffs (C_{F}, C_{F}') are positioned on a garment-facing surface of the at least one first fastening element (FE1) such that substantially the entire surface thereof remains exposed and that the at least one first fastening element (FE1) overlaps said pair of lateral cuffs (C_{F}, C_{F}'). In this embodiment, the cuffs (C_{F}, C_{F}') are typically sandwiched between the topsheet (2) and the at least one first fastening element (FE1). In such a way the surface area available for fastening is maximised.

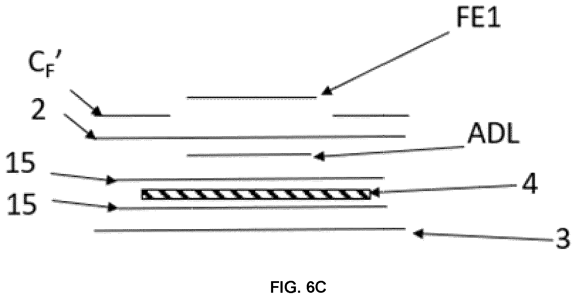

In an embodiment, as illustrated in Fig. 6C, the insert comprises at least a pair of lateral cuffs (C_{F}, C_{F}') wherein at least one of said cuffs is positioned along at least a portion of the first longitudinal edge (8) and at least one other of said cuffs is positioned along at least a portion of the second longitudinal edge (9) such to define a spacing therebetween, and wherein said cuffs (C_{F}, C_{F}') and the at least one first fastening element (FE1) are joined to a body-facing surface of the topsheet (2) with the at least one first fastening element (FE1) being positioned between said cuffs (C_{F}, C_{F}') such that substantially the entire first fastening element (FE1) is exposed along said spacing. Preferably wherein the, typically entire, width (W_{FE1}, W_{FE1'}) of said fastening element(s) lies within said spacing (i.e. the width of the fastening element(s) is less than or equal to the width of said spacing along an axis substantially parallel to the transversal centreline x). In this embodiment, the first fastening element (FE1) may be, preferably only, joined to the topsheet (2) so that it fits between the cuffs (C_{F}, C_{F}') with substantially no overlap. Advantageously, the surface area for fastening is maximised yet reducing the amount of material and cost.

The inserts (1) herein may further comprise longitudinally extending folds (Fₒ, Fₒ') proximal to each of first and second longitudinal edges (8, 9) wherein said folds (Fₒ, Fₒ') comprise a contact surface where a first portion of the backsheet (3) is in contact with a second portion of the backsheet (3) and wherein said contact surface is a garment-facing surface of said backsheet (3). Advantageously this allows to reduce the footprint of the insert as well as importantly create a stiffening structure at the longitudinal edges for ease of handling and coupling to a re-usable outer shell, this without the need of added stiffening structures to be incorporated.

Inserts herein may further comprise an acquisition distribution layer (ADL) positioned between the topsheet and the absorbent core, preferably between the topsheet and the top core wrap layer. The acquisition distribution layer may have a basis weight of from 15 gsm to 55 gsm, preferably from 18 gsm to 50 gsm, even more preferably from 19 gsm to 45 gsm. Preferably the acquisition distribution layer is selected from a carded air-through bonded nonwoven, a carded thermobonded calendered nonwoven, a spunbond nonwoven, and combinations thereof.

Preferably, the insert comprises oppositely disposed longitudinally extending flanges (F_{L}, F_{L}') along the first and second longitudinal edges (8, 9) and wherein said flanges (F_{L}, F_{L}') are folded to form the longitudinally extending folds (Fₒ, Fₒ'), preferably wherein the longitudinally extending flanges (F_{L}, F_{L}') are substantially free of absorbent material. Preferably, the longitudinally extending flanges (F_{L}, F_{L}') comprise the topsheet (2) and the backsheet (3) being directly or indirectly joined to each other (when indirectly joined it preferably means that the acquisition distribution layer and/or portion of cuffs as described herein are interposed between the topsheet and backsheet when the layers are joined together). Advantageously this allows to form stiffening lateral structures whilst minimising material usage.

In an embodiment, the longitudinally extending folds (Fₒ, Fₒ') comprise adhesive joining the longitudinally extending flanges (F_{L}, F_{L}') to a garment-facing surface of the backsheet (3). The adhesive may be continuously or discontinuously disposed along a longitudinal axis extending substantially parallel to the longitudinal centreline (y). Advantageously this allows for further resistance to unfolding which may bring about further benefits especially in terms of mechanical resistance to bending.

Inserts (1) herein may further comprise a wetness indicator that is viewable from a garment-facing side of the backsheet (3) and the insert (1) is joinable to a re-usable outer shell (20) preferably such that the wetness indicator is viewable from a garment-facing side of the re-usable outer shell (20).

Inserts herein preferably comprise a pair of barrier cuffs extending along the first and second longitudinal edges (8, 9) and arranged to provide lateral barriers preventing exudate leakage. Typically, the barrier cuffs comprising a hydrophobic nonwoven and one or more elastics at an apex position thereof such to form standing gathers when the insert is extended for placement within the re-usable outer shell.

Preferably, inserts (1) herein comprise at least a pair of lateral cuffs (C_{F}, C_{F}') wherein at least one of said cuffs is positioned along at least a portion of the first longitudinal edge (8) and at least one of said cuffs is positioned along at least a portion of the second longitudinal edge (9), preferably wherein the cuffs comprise one or more elastics (e, e') proximal to an apex thereof such that a tension thereof causes the cuffs to project upwards away from the topsheet (2). Preferably, the cuffs are positioned substantially inboard of longitudinally extending flanges (F_{L}, F_{L}') but may also be comprised in at least a portion thereof. Advantageously the cuffs not only provide barriers to liquid as generally known in the art but surprisingly synergistically cooperate with the lateral folds to provide a stiffened structure that aids insert location and attachment to a re-usable outer shell. Moreover, better cup formation has been observed when joined to the outer shell and such aiding in better fit and core utilisation when soiling.

Generally, inserts herein are free of transversely extending side panels such as elastic side panels or elastic ears that are rather commonly found in diapers.

Topsheets (2) for use in inserts herein are preferably selected from nonwovens comprising plant-based fibers wherein said plant-based fibers comprise, preferably consist of, harvested fibers other than wood pulp, such that said topsheet (2) has a bio-based content of from about 10% to about 100%, preferably from about 15% to about 100%, even more preferably from about 20% to about 100%, using ASTM D6866-10, method B, and optionally wherein said nonwoven further comprises synthetic fibers generally in an amount to provide added structural integrity such as from 10% to 60% by weight of said nonwoven.

Backsheets for use herein may be breathable and/or comprise a film, preferably polyethylene (PE) based or bio-PE based, and optionally a nonwoven layer with the nonwoven layer being positioned on the outermost surface at a garment facing side of said film. When present, the nonwoven layer may be similar in composition to that described above for the topsheet but may differ in physical parameters such as basis weight, embossments, fiber bonding mechanism and the like.

Preferably, at least 85%, preferably at least 90%wt, more preferably at least 95%wt, by total weight of the disposable absorbent inserts herein (typically all measurements taken in dry/un-used state i.e. on non-soiled inserts) are composed of materials having a bio-based content of from about 10% to about 100%, preferably from about 15% to about 100%, even more preferably from about 20% to about 100%, using ASTM D6866-10, method B. Advantageously the simple insert designs herein enable the use of high bio-based content materials that would otherwise not be possible without compromising at least one of ease of handling, core utilisation performance, acquisition speed, and liquid retention, as described herein.

In an embodiment, each component or layer forming the disposable absorbent inserts herein has a bio-based content of from about 10% to about 100%, preferably from about 15% to about 100%, even more preferably from about 20% to about 100%, using ASTM D6866-10, method B.

The inserts herein may be substantially rectangular in shape but may equally be shaped such as hourglass and/or substantially T-shaped. Other anatomical shapes are further contemplated herein and suitable in the present disclosure.

In an embodiment, the absorbent material comprises, preferably consists of, superabsorbent particles and/or cellulose fibers.

The absorbent cores herein may comprise at least 60%wt of superabsorbent particles, in particular at least 70%wt, preferably at least 80%wt, preferably at least 90%wt, by total weight of the core.

The absorbent inserts herein may comprise a wetness indicator which is visible from the exterior of the insert and/or article and which changes appearance when contacted with a body exudates, in particular urine. The wetness indicator may be placed, when seen from the exterior of the insert and/or article, between the two channel-forming areas and/or channels and/or attachment zones according to some embodiments herein.

The wetness indicator serves the role of alerting the wearer that the insert has been soiled and may need changing.

The wetness indicators for use herein may be according to any wetness indicating system known in the art. It is known that wetness indicator can provide an appearing signal, a disappearing signal or a colour change signal, and of course combinations thereof. An appearing signal will typically not be visible or more generally perceivable in the dry insert, and becomes visible or otherwise perceivable when the insert is wet. An appearing signal may for example be provided by a composition which is transparent or having a colour that matches the colour of the backsheet material, which is typically white, in its dry state, and then changes to a different colour when contacted with urine. Other appearing wetness indicator may also be elements capable of providing a physical sensation indicating a fullness level of the absorbent assembly. Examples of such elements are disclosed in WO2008132630 and include a temperature change element (cooling or heating element), a pressure-inducing element or a foam-producing element.

The wetness indicator composition may be applied to a layer of the absorbent insert as described herein using a conventional technique, for example printing, spraying or coating, during the making of the absorbent insert. The layer may advantageously be the inner surface of the backsheet or the outer surface of the bottom side of the core wrap or both. This allows the wetness indicator to be visible from the exterior of the insert by transparency through the backsheet while keeping the wetness indicator composition within the insert. The wetness indicator may in particular be easily applied on a layer such a nonwoven or film by a slot-coating process especially if the composition is can be applied as a hot-melt. The slot-coating process allows applying a well-defined slot or a series of slots extending in the machine direction of the converting line, which is typically parallel to the longitudinal direction of the insert.

In an embodiment, the absorbent core (4) comprises a core wrap enclosing the absorbent material (5) therein, and wherein the wetness indicator is positioned between a garment-facing side of the core wrap and a body-facing surface of the backsheet (3), preferably positioned between a garment-facing side of the bottom/lower core wrap (15) and a body-facing surface of the backsheet (3).

Preferably, the wetness indicator extends along the longitudinal centerline (y) and may cross the transverse centerline (x), wherein the wetness indicator extends from about the transverse centerline (x) (i.e. from a position proximal to the transverse centerline (x) or a position being on the centeriline (x)) to a first terminal position in a direction towards the first transverse edge (6) and extends from about the transverse centerline (x) to a second terminal position in a direction towards the second transverse edge (7); wherein the distance between the first terminal position and the first transverse edge (6) is less than the distance between the second terminal position and the second transverse edge (7) or vice versa. Advantageously this allows for accurate location/fitting of the insert when a specific front-to-back relationship is required. Indeed the fact that the wetness indicator is visually asymmetrically oriented about the transverse centerline allows for creating indicia for the user on the correct front/back positioning to place onto the corresponding outer shell, especially when the outer shell comprises a corresponding window and/or sensor having substantially the same shape of the wetness indicator which need to be overlayed one on top of the other.

Preferably, the wetness indicator has a first maximum width and the window (23) has a second maximum width (W), generally extending along a direction substantially parallel to the transverse centerline (x), and wherein the first maximum width is less than the second maximum width (W). Advantageously this allows for the full wetness indicator to be viewable from the garment facing side of the outer shell (20) when connected thereto.

Preferably the wetness indicator has a first maximum width being greater than 3mm, preferably from 5mm to 30mm, even more preferably from 10mm to 20mm. Advantageously a wider than normal wetness indicator is advantageous for increased ease of viewing through the outer shell (and/or detection by the optical sensor as described herein), however higher widths do not provide significant improvement in visibility and rather add material and cost.

In an embodiment, the absorbent core (4) comprises one or more attachment zones wherein a top layer of a core wrap (14) is adhered to a lower layer of a core wrap (15) such that one or more channels (13) substantially free of absorbent material are formed, preferably wherein the wetness indicator extends between said channels (13) and preferably overlaps at least a portion of said channels. The channels (13) may extend from 10% to 85%, preferably from 15% to 75%, of a length of the core (4) extending in a direction substantially parallel to the longitudinal axis (y).

Preferably, the absorbent core (4) comprises one or more attachment zones wherein the top layer (14) of the core wrap is adhered to the lower layer (15) of the core wrap such that one or more channels (13) substantially free of absorbent material are formed, preferably inboard of a perimeter of said core (4) so that said channels (13) do not extend to or reach said perimeter. Advantageously this allows to reduce the list of leakage as well as cooperate with the folds to provide improved cup formation when worn.

In a preferred embodiment, channels (13) herein are longitudinally extending and cross both the transverse centerline (x) at a first crossing point and the longitudinal centerline (y) at a second crossing point, wherein the first and second crossing points are different and preferably wherein the second crossing point is positioned closer to the second transverse edge (7) than the first crossing point, and preferably wherein the second transverse edge (7) is positioned proximal to the back region (B) of the outer shell (20) when said insert (1) is connected thereto. Advantageously this allows for improved cup formation in a hybrid concept as well as retaining liquid distribution advantages by guiding exudates to the back of the core.

### THE ABSORBENT ASSEMBLY

Absorbent assemblies herein may comprise an insert (1) as described herein coupled to an outer shell (20) as described herein, the re-usable outer shell (20) generally comprises one or more second fastening elements (FE2) fastenable to, preferably each of, the first fastening element(s) (FE1). Preferably wherein the second fastening elements (FE2) comprise hooks as described herein.

There may be provided a kit of parts comprising: a plurality of inserts (1) as described herein; and one or more outer shells (20) as described herein. Such kits may allow for extended use of the re-usable outer shells.

Preferably, the plurality of inserts (1) differ in at least one of: size, absorbency, biodegradable and/or compostable content, shape, and number of components contained therein. Advantageously this allows for different inserts targeting different types of usage e.g. day vs night, size increases of subjects/wearers etc. whilst retaining substantially the same outer shell.

In any of the above embodiments, the re-usable outer shell (20) may comprise: a front (F) and back (B) waist region having uppermost and lowermost edges; and a crotch region (C) interposed between the front (F) and back (B) waist region, wherein the uppermost edges of the waist region form a waist opening and the lowermost edges of the waist region together with lateral extremities of the crotch region form two oppositely disposed leg openings; wherein at least the body-facing surface of the crotch region (C) is adapted for receiving the absorbent insert (1), and wherein each of the front (F) and/or back (B) waist regions comprise a flap (FP) extending from the upper and/or lowermost edges respectively towards said crotch region (C) along an axis parallel to the longitudinal centerline (y); wherein said flap(s) (FP) overlap the one or more second fastening elements (FE2) that are positioned on a body-facing surface of said re-usable outer shell (20); or wherein said flap(s) (FP) comprise the one or more second fastening elements (FE2) on a garment facing surface thereof.

### METHOD OF MANUFACTURE

Methods for the manufacture of disposable absorbent inserts herein may comprise the steps of:
(i) providing an absorbent core comprising absorbent material therein;
(ii) laminating the absorbent core between a first web of material and a second web and/or film of material, wherein the first web of material forms a topsheet (2) and the second web and/or film of material forms a backsheet (3);
(iii) providing a third web of material, preferably comprising a loop material; and
(iv) applying said third web of material over at least portions of the first web of material corresponding to positions at least proximal and/or adjacent to first and/or second transverse edges (6, 7) of the insert (1) to form least one first fastening element (FE1).

In an embodiment, the third web of material is intermittently applied onto the first web of material such that a separation distance exists therebetween when said third web of material is applied on said first web of material.

Preferably the third web of material is cut into discrete patches of material that are substantially consecutively applied on said first web of material. Said patches generally having a width (in a direction perpendicular to a machine direction) being less than or equal to that of the first web, and a length in the machine direction being less than that of the first web.

In an embodiment, adhesive is applied along substantially the entire width of the discrete patches for joining to the first web of material. An area of the discrete patches adjacent thereto may be left free of adhesive to form an unjoined area extending along the machine direction.

Methods for the manufacture of disposable absorbent inserts herein may comprise the steps of:
(i) providing a first web of material, preferably a liquid permeable nonwoven;
(ii) depositing absorbent material onto said first web;
(iii) providing a second web of material, preferably a liquid permeable nonwoven, and applying said second web of material over the deposited absorbent material, or folding said first web to enclose the deposited absorbent material therein;
(iv) joining the first and second webs together, or the folded first web, at the one or more attachment areas to form an absorbent core comprising absorbent material enclosed by said web(s);
(v) optionally applying an acquisition distribution layer;
(vi) sandwiching the absorbent core, and preferably the acquisition distribution layer, between a liquid permeable layer and a substantially liquid impermeable layer oppositely disposed thereto, preferably with the acquisition distribution layer being in contact with the liquid permeable layer, to form a disposable absorbent insert;
wherein after step (vi) a first folding step is applied wherein first and second longitudinal edges of said insert are tucked-in by folding said edges about a folding axis running parallel to a machine direction such that longitudinally extending folds (Fₒ, Fₒ') proximal to each said first and second longitudinal edges (8, 9) are formed wherein said folds (Fₒ, Fₒ') comprise a contact surface where a first portion of the substantially liquid impermeable layer forming a backsheet (3) is in contact with a second portion of said backsheet (3) and wherein said contact surface is a garment-facing surface of said backsheet (3). Advantageously this method allows for the formation of inserts that reduce the footprint thereof as well as importantly create a stiffening structure at the longitudinal edges for ease of handling and coupling to a re-usable outer shell, this without the need of added stiffening structures to be incorporated.

In an embodiment the folding step is carried out statically (i.e. without the use of rotating or moving elements) and typically comprising one or more stators arranged to fold the respective sections of the insert laminate as it is guided therealong and/or therethrough.

Preferably, after the fist folding step at least a first pressure is applied, for example by one or more pressure rollers. The pressure may be selectively applied substantially only over the folds outboard of a central section comprising the absorbent material, for example by at least two oppositely positioned pressure rollers at a distance from each other along an axis perpendicular to the (z)-axis and parallel to the longitudinal axis (y). Advantageously this allows added consolidating of the folds whilst limiting damage to the absorbent core (e.g. inadvertent crushing of absorbent polymer particles when present or piercing the topsheet and/or backsheet).

In an embodiment, prior to the first folding step an adhesive is applied in one or more continuous or discontinuous stripes along at least a portion of the longitudinally extending flanges (F_{L}, F_{L}'), or an area adjacently inboard thereof, on a garment-facing surface thereof such that, upon folding, the flanges (F_{L}, F_{L}') are joined to the garment-facing surface of the backsheet. In addition or alternatively, during or after the first folding step the folded flanges (F_{L}, F_{L}') are mechanically bonded such that said flanges (F_{L}, F_{L}') are joined to a garment-facing surface of the backsheet at a plurality of discrete bonding joints. Typically such mechanical bonding is selected by heat and/or pressure bonding or ultrasonic bonding. Advantageously this allows for further resistance to unfolding which may bring about further benefits especially in terms of mechanical resistance to bending in yet a cost-effective manner.

In an embodiment, after the first folding step a second folding step is applied wherein the insert is folded about a transverse centerline (x) such that the longitudinally extending folds (Fₒ, Fₒ') substantially enclose other portions of the folded insert therein. In such manner the inserts may then be compacted and effectively stacked for packaging.

Preferably, the method comprises a cutting step after the first folding step to cut the laminated structure into a plurality of disposable absorbent inserts. Preferably wherein the second folding step occurs sequentially after the cutting step.

It is supposed that the present invention is not restricted to any form of realization described previously and that some modifications can be added to the presented embodiments without reappraisal of the appended claims.

## Claims

1. A disposable absorbent insert (1) comprising:
a liquid permeable topsheet (2);
a liquid impermeable backsheet (3); and
an absorbent core (4) comprising absorbent material (5) therein and being sandwiched between said topsheet (2) and backsheet (3);
wherein the insert (1) comprises a perimeter formed by first and second transverse edges (6, 7) and first and second longitudinal edges (8, 9) connecting the first and second transverse edges (6, 7); a longitudinal centerline (y) extending substantially parallel to said first and second longitudinal edges (8, 9) and interposed therebetween such to divide said insert (1) into a first longitudinal half between said longitudinal centerline (y) and said first longitudinal edge (8) and a second longitudinal half between said longitudinal centerline (y) and said second longitudinal edge (9); and a transverse centerline (x) extending substantially parallel to said first and second transverse edges (6, 7) and interposed therebetween such to divide said insert (1) into a first transverse half between said transverse centerline (x) and said first transverse edge (6) and a second transverse half between said transverse centerline (x) and said second transverse edge (7);
**characterised in that** the insert (1) comprises at least one first fastening element (FE1) at a body-facing side of said insert (1), said at least one first fastening element (FE1) being positioned at least proximal to the first and/or second transverse edges (6, 7); and **in that** each said first fastening element (FE1) is configured to secure onto a corresponding second fastening element (FE2) of a re-usable outer shell (20).

2. An insert (1) according to Claim 1 wherein said first fastening element (FE1) comprises a layer that is joined to the topsheet (2) on a body-facing surface thereof, preferably wherein said layer is joined by adhesive along a joining line extending parallel to the first and/or second transverse edges (6, 7).

3. An insert (1) according to any of the preceding Claims comprising at least two first fastening elements (FE1, FE1') joined to the topsheet (2) on a body-facing surface thereof and each being joined at a position proximal to the first or second transverse edges (6, 7) such that said first fastening elements (FE1, FE1') are separated from each other at least along an axis extending substantially parallel to the longitudinal centerline (y).

4. An insert (1) according to any of the preceding Claims wherein first fastening element (FE1), preferably each first fastening element (FE1, FE1'), comprises, preferably consists of, a female fastening element; and the second fastening element (FE2) comprises, preferably consists of, a male fastening element.

5. An insert (1) according to any of the preceding Claims wherein the first fastening element (FE1), preferably each first fastening element (FE1, FE1'), comprises knitted loop material or a nonwoven loop material; and/or wherein the corresponding second fastening element (FE2) comprises hooks.

6. An insert (1) according to any of the preceding Claims wherein the first fastening element(s) (FE1, FE1') have a length (L_{FE1}, L_{FE1'}) extending substantially parallel to the longitudinal centerline (y) and a width (W_{FE1}, W_{FE1'}) extending substantially parallel to the transverse centerline (x), and the length-to-width ratio (L_{FE1}/W_{FE1}, L_{FE1'}/W_{FE1'}) of less than 1, preferably from 0.1 to 0.9, more preferably from 0.2 to 0.8, even more preferably from 0.3 to 0.7.

7. An insert (1) according to any of the preceding Claims wherein the first fastening element(s) (FE1, FE1') are joined to the topsheet (2) at a joining zone (JZ) proximal, preferably adjacent, to the first and/or second transverse edges (6, 7) wherein said joining zone comprises adhesive; and wherein the first fastening element(s) (FE1, FE1') comprise an un-joined zone (UZ) substantially free of adhesive, wherein the un-joined zone (UZ) is positioned adjacent to the joining zone (JZ) and closer to the transverse centerline (x) than said joining zone (JZ).

8. An insert (1) according to Claim 7 wherein a pocket is formed between the topsheet (2) and the first fastening element(s) (FE1, FE1') at the un-joined zone (UZ) to accommodate exudates therein.

9. An insert (1) according to any of the preceding Claims comprising at least a pair of lateral cuffs (C_{F}, C_{F}') wherein at least one of said cuffs is positioned along at least a portion of the first longitudinal edge (8) and at least one other of said cuffs is positioned along at least a portion of the second longitudinal edge (9), preferably wherein the cuffs comprise one or more elastics (e, e') proximal to an apex thereof such that a tension thereof causes the cuffs to project upwards away from the topsheet (2); and wherein said cuffs are joined to a body-facing surface of the first fastening element(s) (FE1, FE1'), or wherein said cuffs are joined to a garment-facing surface of the first fastening element(s) (FE1, FE1') and are at least partly overlapped by said first fastening element(s) (FE1, FE1') such that said cuffs are positioned between the topsheet (2) and said first fastening element(s) (FE1, FE1').

10. An insert (1) according to any of the preceding Claims comprising at least a pair of lateral cuffs (C_{F}, C_{F}') wherein at least one of said cuffs is positioned along at least a portion of the first longitudinal edge (8) and at least one other of said cuffs is positioned along at least a portion of the second longitudinal edge (9) such to define a spacing therebetween, and wherein the at least one first fastening element (FE1) is exposed, preferably only, along said spacing; and/or wherein said cuffs (C_{F}, C_{F}') are positioned on a body-facing surface of the at least one first fastening element (FE1) to define at least two regions where said cuffs (C_{F}, C_{F}') overlap said at least one first fastening element (FE1).

11. An insert (1) according to any of Claims 1 to 9 comprising at least a pair of lateral cuffs (C_{F}, C_{F}') wherein at least one of said cuffs is positioned along at least a portion of the first longitudinal edge (8) and at least one other of said cuffs is positioned along at least a portion of the second longitudinal edge (9) such to define a spacing therebetween, and wherein said cuffs (C_{F}, C_{F}') are positioned on a garment-facing surface of the at least one first fastening element (FE1) such that substantially the entire surface thereof remains exposed and that the at least one first fastening element (FE1) overlaps said pair of lateral cuffs (C_{F}, C_{F}').

12. An insert (1) according to any of Claims 1 to 9 comprising at least a pair of lateral cuffs (C_{F}, C_{F}') wherein at least one of said cuffs is positioned along at least a portion of the first longitudinal edge (8) and at least one other of said cuffs is positioned along at least a portion of the second longitudinal edge (9) such to define a spacing therebetween, and wherein said cuffs (C_{F}, C_{F}') and the at least one first fastening element (FE1) are joined to a body-facing surface of the topsheet (2) with the at least one first fastening element (FE1) being positioned between said cuffs (C_{F}, C_{F}') such that substantially the entire first fastening element (FE1) is exposed along said spacing.

13. An absorbent assembly comprising:
an insert (1) according to any of Claims 1 to 12; and
a re-usable outer shell (20);
wherein the re-usable outer shell (20) comprises one or more second fastening elements (FE2) fastenable to, preferably each of, the first fastening element(s) (FE1).

14. An absorbent assembly according to Claim 13 wherein the re-usable outer shell (20) comprises:
a front (F) and back (B) waist region having uppermost and lowermost edges; and
a crotch region (C) interposed between the front (F) and back (B) waist region,
wherein the uppermost edges of the waist region form a waist opening and the lowermost edges of the waist region together with lateral extremities of the crotch region form two oppositely disposed leg openings;
wherein at least the body-facing surface of the crotch region (C) is adapted for receiving the absorbent insert (1), and wherein each of the front (F) and/or back (B) waist regions comprise a flap (FP) extending from the upper and/or lowermost edges respectively towards said crotch region (C) along an axis parallel to the longitudinal centerline (y);
wherein said flap(s) (FP) overlap the one or more second fastening elements (FE2) that are positioned on a body-facing surface of said re-usable outer shell (20); or wherein said flap(s) (FP) comprise the one or more second fastening elements (FE2) on a garment facing surface thereof.

15. A kit of parts comprising:
a plurality of inserts (1) according to any of Claims 1 to 12 for an absorbent assembly according to any of Claims 13 to 14, wherein the plurality of inserts (1) differ in at least one of: size, absorbency, biodegradable and/or compostable content, shape, and number of components contained therein.

16. A method for the manufacture of a disposable absorbent insert (1), preferably according to any of Claims 1 to 12, said method comprising the steps of:
(i) providing an absorbent core comprising absorbent material therein;
(ii) laminating the absorbent core between a first web of material and a second web and/or film of material, wherein the first web of material forms a topsheet (2) and the second web and/or film of material forms a backsheet (3);
(iii) providing a third web of material, preferably comprising a loop material or a nonwoven comprising an embossment pattern; and
(iv) applying said third web of material over at least portions of the first web of material corresponding to positions at least proximal to first and/or second transverse edges (6, 7) of the insert (1) to form least one first fastening element (FE1).
